# EUROPEAN PATENT APPLICATION

(11) **EP 2 260 847 A1**
(43) Date of publication of application: **15.12.2010**
(21) Application number: 09727847.7
(22) Date of filing: 30.03.2009
(51) Int. Cl.: A61K 31/496, A61P 27/02, A61P 31/04

(54) **AQUEOUS LIQUID CONTAINING GATIFLOXACIN**

(30) Priority: 31.03.2008 US 41199
(71) Applicant: Kyorin Pharmaceutical Co., Ltd., Tokyo 101-8311 (JP); Senju Pharmaceutical Co., Ltd., Osaka-shi, Osaka 541-0046 (JP)
(72) Inventor: SAWA, Shirou, Kobe-shi Hyogo 651-2241 (JP)
(74) Representative: Gillard, Richard Edward
(86) International application number: PCT/JP2009/056474
(87) International publication number: WO 2009/123099

(57) **Abstract**

There is provided an aqueous liquid preparation comprising Gatifloxacin or a pharmacologically acceptable salt thereof or a hydrate thereof, phosphoric acid or a salt thereof, and xanthan gum, wherein a pH thereof is 5.5 or more and less than 7.0. The aqueous liquid preparation has improved intraocular penetration of Gatifloxacin. Further, the formation of a precipitate during storage at a lower temperature and at the time of freezing and thawing of the aqueous liquid preparation is suppressed by incorporating at least one of the ingredient selected from the group consisting of nicotinamide, caffeine, methylglucamine, methyl parahydroxybenzoate and a salt thereof into the aqueous liquid preparation.

## Description

### BACKGROUND OF THE INVENTION

### Technical Field

The present invention relates to a Gatifloxacin-containing aqueous liquid preparation.

### Background Art

Gatifloxacin is a new quinolone synthetic antimicrobial and exhibits strong antimicrobial activity against Gram-positive bacteria, anaerobic bacteria and mycoplasma, not to mention Gram-negative bacteria, and is marketed as eyedrops for treating bacterial conjunctivitis in the ophthalmologic field. In general, when a drug is administered by instillation, intraocular penetration of the drug is low. Therefore, eyedrops having improved intraocular penetration of a drug and an enhanced therapeutic effect of a drug has been required.

U.S. Patent No. 4,136,177 discloses, as a technique for intraocular penetration of a drug, an aqueous composition containing an ophthalmologic drug and xanthan gum, and also describes that xanthan gum enhances a therapeutic effect of Ecothiopate. Since a precipitate is likely to be formed when mixed with xanthan gum and fluoroquinolone, JP 2003-513046 A (corresponding U.S. Patent No. 6,331,540) discloses a technique in which formation of a precipitate is suppressed by mixing with a water-soluble calcium salt.

U.S. Patent No. 6,333,045 discloses, as a technique of enhancing cornea permeability of a drug, an aqueous liquid preparation containing Gatifloxacin or a salt thereof at a relatively low concentration of Gatifloxacin of lower than 0.5 w/v%, and sodium edetate at a low concentration. Furthermore, U.S. Patent No. 6,333,045 reports that the solubility of Gatifloxacin can be increased and precipitation of Gatifloxacin crystals can be prevented in an aqueous liquid preparation containing Gatifloxacin at a relatively low concentration of lower than 0.5 w/v% or a salt thereof by adding sodium edetate.

Some reports published prior to U.S. Patent No. 6,333,045 mention that cornea permeability of a drug by sodium edetate depends on the concentration of sodium edetate, and sodium edetate at a relatively high concentration of 0.5% is required so as to enhance cornea permeability of the drug (Journal of Pharmaceutical Science, 77: 3-14, 1988; Investigative Ophthalmology & Visual Science, 26: 110-113, 1985; Experimental Eye Research, 54: 747-757, 1992; Pharmaceutical Research, 12, 8: 1146-1150, 1995).

Further, it has also been known that there are some reports mentioning that the use of sodium edetate has such a problem as irritation to the cornea upon instillation (Pharmaceutical Research, 15, 8: 1275-1279, 1998; Drugs and Pharmaceutical Sciences, 58, Ophthalmic Drug Delivery Systems: 188).

The disclosures of the above-mentioned documents are incorporated by reference herein.

### SUMMARY OF THE INVENTION

One object of the present invention is to improve intraocular penetration of Gatifloxacin of an aqueous liquid preparation comprising Gatifloxacin or a pharmacologically acceptable salt thereof or a hydrate thereof.

Another object of the present invention is to suppress the formation of a precipitate during storage at a lower temperature and at the time of freezing and thawing of the aqueous liquid preparation.

These and other objectives as well as advantages of the present invention will become apparent to those skilled in the art from the following description.

That is, the present invention relates to:
(1) An aqueous liquid preparation comprising Gatifloxacin or a pharmacologically acceptable salt thereof or a hydrate thereof, phosphoric acid or a salt thereof, and xanthan gum, wherein the pH is 5.5 or more and less than 7.0;
(2) The aqueous liquid preparation according to the above (1), wherein the preparation contains 0.45 to 2 w/v% of Gatifloxacin or a pharmacologically acceptable salt thereof or a hydrate thereof as free Gatifloxacin;
(3) The aqueous liquid preparation according to the above (1) or (2), wherein the preparation contains at least 0.6 w/v% or more of phosphoric acid or a salt thereof;
(4) The aqueous liquid preparation according to the above (1) or (2), wherein the preparation contains 0.6 to 1.9 w/v% of phosphoric acid or a salt thereof;
(5) The aqueous liquid preparation according to any one of the above (1) to (4), wherein the preparation further contains sodium chloride;
(6) The aqueous liquid preparation according to any one of the above (1) to (5), wherein the preparation further contains at least one of the ingredient selected from the group consisting of nicotinamide, caffeine, methylglucamine, methyl parahydroxybenzoate and a salt thereof;
(7) The aqueous liquid preparation according to any one of the above (1) to (6), which is eyedrops; and
(8) A method for suppressing the formation of a precipitate during storage at a lower temperature and at the time of freezing and thawing of an aqueous liquid preparation comprising Gatifloxacin or a pharmacologically acceptable salt thereof or a hydrate thereof, phosphoric acid or a salt thereof, and xanthan gum, whose pH is 5.5 or more and less than 7.0, which comprises incorporating at least one of the ingredient selected from the group consisting of nicotinamide, caffeine, methylglucamine, methyl parahydroxybenzoate and a salt thereof into the aqueous liquid preparation.

According to the present invention, intraocular penetration of Gatifloxacin can be improved by adding phosphoric acid or a salt thereof and xanthan gum to an aqueous liquid preparation comprising Gatifloxacin or a pharmaceutically acceptable salt thereof or a hydrate thereof.

Further, according to the present invention, intraocular penetration of Gatifloxacin can be improved by adding phosphoric acid or a salt thereof and xanthan gum to an aqueous liquid preparation comprising Gatifloxacin or a pharmacologically acceptable salt thereof or a hydrate thereof as free Gatifloxacin, for example, 0.45 to 2 w/v%, preferably 0.45 to 1.5 w/v%, particularly preferably 0.7 to 1.2 w/v%.

Further, according to the present invention, intraocular penetration of Gatifloxacin can be improved by adding phosphoric acid or a salt thereof and xanthan gum without adding sodium edetate.

Furthermore, according to the present invention, intraocular penetration of Gatifloxacin can be enhanced by adding a small amount of sodium edetate to a formulation of phosphoric acid or a salt thereof and xanthan gum.

Furthermore, according to the present invention, intraocular penetration of Gatifloxacin can be improved by adding phosphoric acid or a salt thereof and xanthan gum to an aqueous liquid preparation comprising Gatifloxacin or a pharmacologically acceptable salt thereof or a hydrate thereof as free Gatifloxacin, for example, 0.45 to 2 w/v%, preferably 0.45 to 1.5 w/v%, particularly preferably 0.7 to 1.2 w/v% without adding sodium edetate.

Moreover, according to the present invention, intraocular penetration of Gatifloxacin can be enhanced by adding a small amount of sodium edetate to a formulation of phosphoric acid or a salt thereof and xanthan gum in an aqueous liquid preparation comprising Gatifloxacin or a pharmacologically acceptable salt thereof or a hydrate thereof as free Gatifloxacin, for example, 0.45 to 2 w/v%, preferably 0.45 to 1.5 w/v%, particularly preferably 0.7 to 1.2 w/v%.

In addition, according to the present invention, the formation of a precipitate during storage at a lower temperature and at the time of freezing and thawing of an aqueous liquid preparation comprising Gatifloxacin or a pharmacologically acceptable salt thereof or a hydrate thereof, phosphoric acid or a salt thereof and xanthan gum is suppressed by incorporating at least one of the ingredient selected from the group consisting of nicotinamide, caffeine, methylglucamine, methyl parahydroxybenzoate and a salt thereof into the aqueous liquid preparation.

### DETAILED DESCRIPTION OF THE PREFERRED EMBODIMENTS

The chemical name of Gatifloxacin is (±)-1-cyclopropyl-6-fluoro-1,4-dihydro-8-methoxy-7-(3-methyl-1-piperazinyl)-4-oxo-3-quinolinecarboxylic acid.

Examples of the pharmacologically acceptable salt of Gatifloxacin include salts with inorganic acids such as hydrochloric acid, sulfuric acid, and phosphoric acid; salts with organic acids such as methanesulfonic acid, lactic acid, oxalic acid, and acetic acid; and salts with sodium, potassium, magnesium, calcium, aluminum, cerium, chromium, cobalt, copper, iron, zinc, platinum, and silver. Examples of the hydrate include 2/5, 1/2, 3/2, and 5 hydrates.

The content of Gatifloxacin or a pharmacologically acceptable salt thereof or a hydrate thereof in the aqueous liquid preparation is preferably from 0.45 to 2 w/v%, more preferably from 0.45 to 1.5 w/v%, particularly preferably from 0.7 to 1.2 w/v%, in terms of free Gatifloxacin in view of intraocular penetration and stability of the pharmaceutical preparation.

Examples of the phosphoric acid or a salt thereof include phosphoric acid, sodium dihydrogenphosphate, sodium hydrogenphosphate, trisodium phosphate, potassium dihydrogenphosphate, and dipotassium phosphate. Hydrates thereof can also be used. Among these, sodium dihydrogenphosphate or a hydrate thereof is preferred. The amount of phosphoric acid or a salt thereof in the aqueous liquid preparation is preferably at least 0.5 w/v%, preferably from 0.6 to 3 w/v%, more preferably from 0.6 to 2.2 w/v%, still more preferably from 0.6 to 1.9 w/v%, particularly preferably from 0.7 to 1.6 w/v%, in view of suppression of production of a precipitate upon freeze-thawing.

The average molecular weight of xanthan gum is usually from 100,000 to 50,000,000, preferably from 200,000 to 20,000,000, particularly preferably from 1,000,000 to 10,000,000. The amount of xanthan gum is usually from about 0.1 to 0.5 w/v%.

The amount of sodium chloride in the aqueous liquid preparation is preferably 0.2 w/v% or more, more preferably from 0.2 to 1.8 w/v%, still more preferably from 0.2 to 1 w/v%.

The pH of the aqueous liquid preparation is usually 5.5 or more and less than 7, preferably from 5.6 to 6.9, more preferably from 5.8 to 6.6.

Nicotinamide, caffeine, methylglucamine, methyl parahydroxybenzoate and a salt thereof can be used alone or by combining two or more thereof. Examples of a salt of methyl parahydroxybenzoate include sodium salt, potassium salt and the like. The amount of these ingredients is at least 0.01 w/v%, preferably from 0.02 to 3 w/v%, more preferably from 0.05 to 1 w/v%.

To the aqueous liquid preparation of the present invention, isotonizing agents (for example, potassium chloride, boric acid, glycerin, propylene glycol, mannitol, sorbitol, glucose, etc.), preservatives (benzalkonium chloride, benzethonium chloride, chlorhexidine gluconate, chlorobutanol, benzyl alcohol, sodium dehydroacetate, parahydroxybenzoate esters, etc.), viscosity agents (methyl cellulose, hydroxyethyl cellulose, hydroxypropylmethyl cellulose, carboxymethyl cellulose, sodium hyaluronate, carboxyvinyl polymer, polyvinyl alcohol, polyvinyl pyrrolidone, macrogol, etc.), pH adjustors (hydrochloric acid, sodium hydroxide, acetic acid, phosphoric acid, etc.), and stabilizing agents (sodium edetate, citric acid, etc.) can be appropriately added, if necessary. When sodium edetate is added, the content is preferably from 0.01 to 0.1 w/v%.

The aqueous liquid preparation of the present invention can be produced by a technique known in the art such as dispersion and dissolution of the desired ingredients in the form suitable for ophthalmic topical administration, preferably eyedrops.

In the present invention, "lower temperature" means temperature of around 4°C, preferably 4°C ± 1°C.

Hereinafter, the following Test Examples and Examples further illustrate the present invention in detail but are not to be construed to limit the scope thereof.

### Test Example 1: Penetration Test of Gatifloxacin to Tissue

### (Preparation of Test Pharmaceutical Preparation)

In accordance with the formulation shown in Table 1, Gatifloxacin-containing eyedrops of Examples 1 to 3 and Comparative Examples 1 to 4 were prepared according to a conventional method.

**Table 1**

| Formulation (w/v%) | Example 1 | Example 2 | Examp le 3 | Comparative Example 1 | Comparative Example 2 | Comparative Example 3 | Comparative Example 4 |
|---|---|---|---|---|---|---|---|
| Gatifloxacin 3/2 hydrate | 0.5 | 0.75 | 1.0 | 0.5 | 0.75 | 1.0 | 0.75 |
| Sodium dihydrogen -phosphate dihydrate | 0.8 | 0.8 | 1.0 | 0.8 | 0.8 | 1.0 | - |
| Boric acid | - | - | - | - | - | - | 1.6 |
| Xanthan gum | 0.2 | 0.2 | 0.2 | - | - | - | 0.2 |
| Sodium chloride | 0.55 | 0.55 | 0.55 | 0.55 | 0.55 | 0.55 | 0.7 |
| Hydrochloric acid/ Sodium hydroxide | q.s. | q.s. | q.s. | q.s. | q.s. | q.s. | q.s. |
| Purified water | q.s. | q.s. | q.s. | q.s. | q.s. | q.s. | q.s. |
| PH | 6.0 | 6.0 | 6.0 | 6.0 | 6.0 | 6.0 | 6.0 |

As xanthan gum, ECHO GUM T manufactured by Dainippon Sumitomo Pharma Co., Ltd. was used.

### (Test Procedure)

To Japanese white male rabbits, each having a weight of about 2.5 kg, 50 µL of the Gatifloxacin-containing eyedrops of each formulation were administered by instillation once. Five minutes after instillation, tear was collected using a capillary (MICROCAPS 2 µL, Drummond). 0.5 hour after instillation, rabbits were euthanized by excessive administration of 5% pentobarbital sodium. The anterior eye part was washed with physiological saline and the aqueous humor was aspirated by a syringe with a 27 Gage injection needle, and then the conjunctiva was removed using scissors.

The aqueous humor was filtered through a filter (0.2 µm) to obtain a sample solution. After measuring the weight of the tear, it was mixed with 0.3 mL of a mobile phase and then filtered through a filter (0.2 µm) to obtain a sample solution. After measuring the weight of the conjunctiva, 5 mL of acetonitrile was added and the conjunctiva was chopped, followed by shaking (at 200 rpm for 20 minutes) and centrifugal separation (at 2,000 rpm for 10 minutes). The supernatant (4 mL) was separated and dried under reduced pressure, then dissolved in 0.5 mL of a mobile phase and filtered through a filter (0.2 µm) to obtain a sample solution. With respect to 50 µL of the sample solution, the concentration of free Gatifloxacin in the tissue was measured under the following HPLC conditions (aqueous humor: n = 3, conjunctiva: n = 3, tear: n = 2).

### (HPLC Measurement Conditions)

Detector: Ultraviolet absorptiometer (measuring wavelength: 280 nm)
Column: Inertsil ODS-3, 4.6 mmΦ × 150 mm. GL Sciences Inc. Gard Column: Inertsil ODS-3, 4.6 mmΦ × 5 mm, Cartridge type, GL Sciences Inc.
Column temperature: 40°C
Mobile phase: 180 mL of acetonitrile, 810 mL of water and 10 mL of triethylamine were mixed and the pH was adjusted to 4.5 using phosphoric acid.
Flow rate: 0.8 mL/min
Injection amount: 50 µL

### (Results)

Table 2 shows the concentration of free Gatifloxacin in each tissue after instillation of the eyedrops of Examples 1 to 3 and Comparative Examples 1 to 4.

**Table 2**

| Formulation | Concentration of free Gatifloxacin | | |
|---|---|---|---|
| | Concentration in aqueous humor (µg/mL) | Concentration in conjunctiva (µg/g) | Concentration in tear (µg/mL) |
| Example 1 | 1.107 ± 0.412 | 4.697 ± 0.939 | 2519.20 ± 1077.02 |
| Example 2 | 1.461 ± 0.568 | 3.716 ± 0.694 | 3932.64 ± 757.76 |
| Example 3 | 1.767 ± 0.072 | 7.506 ± 1.Q98 | 6627.52 ± 1430.33 |
| Comparative Example 1 | 0.657 ± 0.123 | 2.136 ± 0.222 | 862.76 ± 764.58 |
| Comparative Example 2 | 1.037 ± 0.137 | 2.284 ± 1.084 | 2016.14 ± 897.73 |
| Comparative Example 3 | 1.227 ± 0.031 | 2.670 ± 0.570 | 2381.92 ± 2588.81 |
| Comparative Example 4 | 0.879 ± 0.113 | - | - |

| | | | |
|---|---|---|---|
| Each value shows an average ± standard deviation. | | | |

As shown in Table 2, the eyedrops containing xanthan gum of Examples 1 to 3 show higher concentration of any tissue of aqueous humor, conjunctiva and tear as compared with the eyedrops containing no xanthan gum of Comparative Examples 1 to 3. The eyedrops containing sodium dihydrogenphosphate of Example 2 show higher concentration of Gatifloxacin in the aqueous humor as compared with the eyedrops containing boric acid of Comparative Example 4. As is apparent from these results, intraocular penetration of Gatifloxacin is improved by adding xanthan gum and phosphoric acid.

### Test Example 2: Penetration Test of Gatifloxacin to Tissue

### (Test Pharmaceutical Preparation)

Gatifloxacin-containing eyedrops of Example 2, Comparative Example 2 and Comparative Example 4 of Test Example 1 were used.

### (Test Procedure)

To Japanese white male rabbits, each having a weight of about 2.5 kg, 50 µL of the Gatifloxacin-containing eyedrops of each formulation were administered by instillation once. Five minutes, 0.5 hour, 1 hour, 2 hours and 4 hours after instillation, tears were collected using a capillary (MICROCAPS 2 µL, Drummond). Rabbits were euthanized by excessive administration of 5% pentobarbital sodium 0.5 hour, 1 hour, 2 hours, 4 hours, 6 hours and 12 hours after instillation. The anterior eye part was washed with physiological saline and then the aqueous humor was aspirated by a syringe with a 27 Gage injection needle, and the conjunctiva was removed using scissors.

The aqueous humor was filtered through a filter (0.2 µm) to obtain a sample solution. After measuring the weight of the tear, it was added with 0.3 mL of a mobile phase and then filtered through a filter (0.2 µm) to obtain a sample solution. After measuring the weight of the conjunctiva, 5 mL of acetonitrile was added and the conjunctiva was chopped, followed by shaking (at 200 rpm for 20 minutes) and centrifugal separation (at 2,000 rpm for 10 minutes). The supernatant (4 mL) was separated and dried under reduced pressure, then dissolved in 0.5 mL of a mobile phase and filtered through a filter (0.2 µm) to obtain a sample solution. With respect to 50 µL of the sample solution, the concentration of free Gatifloxacin in the tissue was measured under the same HPLC conditions as in Test Example 1 (aqueous humor: n = 3, conjunctiva: n = 3, tear: n = 5 to 7).

### (Results)

Table 3 shows the concentration of free Gatifloxacin in the aqueous humor, measured 0.5 to 12 hours after instillation of the eyedrops of Example 2, Comparative Example 2 and Comparative Example 4.

**Table 3**

| Time (hours) | Concentration of free Gatifloxacin in aqueous humor (µg/mL) | | |
|---|---|---|---|
| | Example 2 | Comparative Example 2 | Comparative Example 4 |
| 0.5 | 1.549 ± 0.115 | 0.932 ± 0.200 | 0.879 ± 0.113 |
| 1 | 2.031 ± 0.047 | 1.146 ± 0.213 | 1.562 ± 0.434 |
| 2 | 2.591 ± 1.337 | 1.277 ± 0.582 | 1.493 ± 0.556 |
| 4 | 0.737 ± 0.284 | 0.347 ± 0.173 | 0.386 ± 0.056 |
| 6 | 0.228 ± 0.154 | 0.116 ± 0.024 | 0.124 ± 0.054 |
| 12 | 0.019 ± 0.003 | 0.013 ± 0.012 | 0.021 ± 0.004 |

| | | | |
|---|---|---|---|
| Each value shows an average ± standard deviation. | | | |

As shown in Table 3, the eyedrops of Example 2 wherein xanthan gum has been added show improve penetration of Gatifloxacin to the aqueous humor and sustain the effect for a long time as compared with the eyedrops of Comparative Example 2 wherein no xanthan gum has been added.

The eyedrops of Example 2 wherein sodium dihydrogenphosphate has been added are excellent in penetration of Gatifloxacin to the aqueous humor and the prolonged effect as compared with the eyedrops of Comparative Example 4, wherein boric acid has been added.

Table 4 shows the area under the drug concentration vs. time curve (AUC (0-12h)) of free Gatifloxacin in the aqueous humor, until 12 hours have passed since instillation, of the eyedrops of Example 2, Comparative Example 2 and Comparative Example 4, and the time during which a minimal inhibitory concentration (MIC) of free Gatifloxacin in the aqueous humor (T>MIC) is maintained. Regarding T>MIC, *Enterococcus faecalis* (ophthalmologic clinical isolated strain A-2-7, MIC = 0.39 µg/mL) was used as an indicator.

**Table 4**

| Formulation | Example 2 | Comparative Example 2 | Comparative Example 4 |
|---|---|---|---|
| AUC (0 → 12 h) (µg•h/mL) | 8.63 | 4.44 | 5.18 |
| T > MIC (h) | 5.24 | 3.70 | 3.77 |

As shown in Table 4, the eyedrops of Example 2 wherein xanthan gum and sodium dihydrogenphosphate have been added show AUC increased by 1.9 times as compared with the eyedrops of Comparative Example 2 wherein no xanthan gum has been added, and show AUC increased by 1.7 times as compared with the eyedrops of Comparative Example 4 wherein no sodium dihydrogenphosphate has been added.

The eyedrops of Example 2 wherein xanthan gum and sodium dihydrogenphosphate have been added show T>MIC increased by 1.4 times as compared with the eyedrops of Comparative Example 2 wherein no xanthan gum has been added, and show T>MIC increased by 1.4 times as compared with the eyedrops of Comparative Example 4 wherein no sodium dihydrogenphosphate has been added.

Table 5 shows the concentration of free Gatifloxacin in the conjunctiva, measured 0.5 to 6 hours after instillation, of the eyedrops of Example 2 and Comparative Example 2.

**Table 5**

| Time (hours) | Concentration of free Gatifloxacin in conjunctiva (µg/g) | |
|---|---|---|
| | Example 2 | Comparative Example 2 |
| 0.5 | 3.716 ± 0.594 | 2.284 ± 1.084 |
| 1 | 2.320 ± 0.679 | 1.393 ± 0.778 |
| 2 | 1.364 ± 0.743 | 0.565 ± 0.167 |
| 4 | 0.433 ± 0.206 | 0.329 ± 0.175 |
| 6 | 0.255 ± 0.044 | 0.232 ± 0.027 |

Each value shows an average ± standard deviation.

As shown in Table 5, the eyedrops of Example 2 wherein xanthan gum has been added show improved penetration of Gatifloxacin to the conjunctiva and sustain the effect for a long time as compared with the eyedrops of Comparative Example 2 wherein no xanthan gum has been added.

Table 6 shows the area under the drug concentration vs. time curve (AUC (0→6h)) of free Gatifloxacin in the conjunctiva, until 6 hours have passed since instillation, of the eyedrops of Example 2 and Comparative Example 2.

**Table 6**

| Formulation AUC | Example 2 | Comparative Example 2 |
|---|---|---|
| (0 → 6 h) (µg•h/mL) | 6 | 3.92 |

As shown in Table 6, the eyedrops of Example 2 wherein xanthan gum has been added show AUC increased by 1.7 times as compared with the eyedrops of Comparative Example 2 wherein no xanthan gum has been added.

Table 7 shows the concentration of Gatifloxacin in the tear, measured 5 minutes to 4 hours after instillation, of the eyedrops of Example 2 and Comparative Example 2.

**Table 7**

| Time | Concentration of free Gatifloxacin in tears (µg/mL) | |
|---|---|---|
| | Example 2 | Comparative Example 2 |
| 5 minutes | 3932.64 ± 757.76 | 2016.14 ± 897.73 |
| 0.5 hour | 132.26 ± 123.76 | 14.48 ± 11.05 |
| 1 hour | 25.72 ± 46.75 | 4.85 ± 2.91 |
| 2 hours | 2.20 ± 1.61 | 4.24 ± 5.07 |
| 4 hours | 0.99 ± 1.36 | 0 |

| | | |
|---|---|---|
| Each value shows an average ± standard deviation. | | |

As shown in Table 7, the eyedrops of Example 2 wherein xanthan gum has been added show increased concentration of Gatifloxacin in the tear as compared with the eyedrops of Comparative Example 2 wherein no xanthan gum has been added.

### Test Example 3: Freeze-Thawing Test

### (Test Procedure)

In accordance with the formulation shown in Table 8, Gatifloxacin-containing aqueous liquid preparations of Reference Examples 1 to 4 were prepared by a conventional method. Each aqueous liquid preparation was filled into a glass ampoule, followed by storage in a freezer at -30°C. The frozen aqueous liquid preparation was thawed by returning the temperature to room temperature. The Gatifloxacin-containing aqueous liquid preparations wherein a precipitate was produced were strongly shaken. This freeze-thawing operation was repeated 10 times and the description of the preparations was then visually observed. In accordance with the following criteria, formation of a precipitate was judged.

### (Judgment of Formation of Precipitate)

-: Foreign insoluble matter was not observed and the description did not vary.
++: Foreign insoluble matter was remarkably generated.

**Table 8**

| Formulation (w/v%) | Reference Example 1 | Reference Example 2 | Reference Example 3 | Reference Example 4 |
|---|---|---|---|---|
| Gatifloxacin 3/2 hydrate | 0.75 | 0.75 | 0.75 | 0.75 |
| Sodium dihydrogenphosphate dihydrate | 0.8 | 0.8 | 0.8 | - |
| Sodium citrate | - | - | - | 0.8 |
| Sodium chloride | 0.2 | 0.2 | 0.2 | 0.2 |
| Hydrochloric acid/Sodium hydroxide | q.s. | q.s. | q.s. | q.s. |
| Purified water | q.s. | q.s. | q.s. | q.s. |
| pH | 6.0 | 6.5 | 7.0 | 6.5 |
| Production of precipitate | - | - | ++ | ++ |

### (Results)

The formation of a precipitate upon freeze-thawing of the Gatifloxacin-containing aqueous liquid preparation was suppressed by adding sodium dihydrogenphosphate to the Gatifloxacin-containing aqueous liquid preparation thereby adjusting the pH to less than 7. On the other hand, in case where the pH was adjusted to 7 (Reference Example 3) and sodium citrate was added in place of sodium dihydrogenphosphate (Reference Example 4), severe foreign insoluble matter was formed upon freeze-thawing.

### Test Example 4: Freeze-Thawing Test

### (Test Procedure)

In accordance with the formulation shown in Table 9, Gatifloxacin-containing aqueous liquid preparations of Reference Examples 5 to 9 were prepared by a conventional method. Each aqueous liquid preparation was filled into a glass ampoule, followed by storage in a freezer at -30°C. The frozen aqueous solution was thawed by returning the temperature to room temperature. The Gatifloxacin-containing aqueous liquid preparation wherein a precipitate was formed were strongly shaken. This freeze-thawing operation was repeated 3 times and the description of the preparation was then visually observed. In accordance with the following criteria, formation of a precipitate was judged.

### (Judgment of Formation of Precipitate)

-: Foreign insoluble matter was not observed and the description did not vary.
+: Foreign insoluble matter was observed and the description varied.
++: Foreign insoluble matter was remarkably generated.

**Table 9**

| Formulation (w/v%) | Reference Example 5 | Reference Example 6 | Reference Example 7 | Reference Example 8 | Reference Example 9 |
|---|---|---|---|---|---|
| Gatifloxacin 3/2 hydrate | 0.75 | 0.75 | 0.75 | 0.75 | 0.75 |
| Sodium dihydrogen-phosphate dihydrate | 0.5 | 1.0 | 1.5 | 2.0 | 2.0 |
| Sodium chloride | - | - | - | - | 0.2 |
| Hydrochloric acid/Sodium hydroxide | q.s. | q.s. | q.s. | q.s. | q.s. |
| Purified water | q.s. | q.s. | q.s. | q.s. | q.s. |
| pH | 6.5 | 6.5 | 6.5 | 6.5 | 6.5 |
| Formation of precipitate | + | - | - | ++ | - |

In the Gatifloxacin-containing aqueous liquid preparations of Reference Examples 6 and 7 wherein 1.0 to 1.5% of sodium dihydrogenphosphate dihydrate has been added, a foreign insoluble matter and a variation in the description were not observed upon freeze-thawing. On the other hand, in the Gatifloxacin-containing aqueous liquid preparation of Reference Example 5 wherein 0.5% of sodium dihydrogenphosphate dihydrate has been added, a foreign insoluble matter and a variation in description were observed. In the Gatifloxacin-containing aqueous liquid preparation of Reference Example 8 wherein 2.0% of sodium dihydrogenphosphate dihydrate has been added, a foreign insoluble matter was severely observed. In the Gatifloxacin-containing aqueous liquid preparation of Reference Example 9 wherein 2.0% of sodium dihydrogenphosphate dihydrate and 0.2% of sodium chloride have been added, a foreign insoluble matter was not observed and the description did not vary.

### Preparation Example

In accordance with the formulation shown in Table 10, a Gatifloxacin-containing eyedrops are prepared by a conventional method.

**Table 10**

| Formulation (w/v%) | Example 4 | Example 5 | Example 6 | Example 7 | Example 8 | Example 9 |
|---|---|---|---|---|---|---|
| Gatifloxacin 3/2 hydrate | 0.75 | 0.75 | 0.75 | 0.75 | 0.75 | 1.0 |
| Sodium dihydrogenphosphate dihydrate | 0.8 | 0.8 | 0.8 | 0.8 | 0.8 | 1.0 |
| Xanthan gum | 0.2 | 0.2 | 0.2 | 0.2 | 0.2 | 0.2 |
| Sodium chloride | 0.55 | 0.55 | 0.9 | 0.9 | 0.75 | 0.9 |
| Benzalkonium chloride | 0.005 | 0.0025 | 0.005 | 0.0025 | - | - |
| Sodium edetate | 0.01 | 0.01 | 0.01 | 0.01 | - | - |
| Hydrochloric acid/Sodium hydroxide | q.s. | q.s. | q.s. | q.s. | q.s. | q.s. |
| Purified water | q.s. | q.s. | q.s. | q.s. | q.s. | q.s. |
| pH | 6.0 | 6.0 | 6.0 | 6.0 | 6.0 | 6.0 |

| | | | | | | |
|---|---|---|---|---|---|---|
| As xanthan gum, ECHO GUM T manufactured by Dainippon Sumitomo Pharma Co., Ltd. was used. | | | | | | |

The Gatifloxacin-containing eyedrops of Example 4 (5 mL each) were filled into a colorless PP container and a colorless PE container, followed by storage at 60°C for 4 weeks. As a result, description, pH and Gatifloxacin content did not vary and the Gatifloxacin-containing eyedrops were stable.

### Test Example 5: Penetration Test of Aqueous Humor

### (Preparation of Test Pharmaceutical Preparation)

In accordance with the formulation shown in Table 11, Gatifloxacin-containing eyedrops of Comparative Example 3 and Examples 10 to 13 were prepared according to a conventional method.

**Table 11**

| Formulation (w/v%) | Comparative Example 3 | Example 10 | Example 11 | Example 12 | Example 13 |
|---|---|---|---|---|---|
| Gatifloxacin 3/2 hydrate | 0.75 | 0.75 | 0.75 | 0.75 | 0.75 |
| Sodium dihydrogenphosphate dihydrate | 0.8 | 0.8 | 0.8 | 0.8 | 0.8 |
| Xanthan gum | - | 0.1 | 0.2 | 0.35 | 0.5 |
| Sodium chloride | 0.55 | 0.55 | 0.55 | 0.55 | 0.55 |
| Hydrochloric acid | q.s. | q.s. | q.s. | q.s. | q.s. |
| Purified water | q.s. | q.s. | q.s. | q.s. | q.s. |
| PH | 6.0 | 6.0 | 6.0 | 6.0 | 6.0 |

As xanthan gum, ECHO GUM T manufactured by Dainippon Sumitomo Pharma Co., Ltd. was used.

### (Test Procedure)

To Japanese white male rabbits, each having a weight of 2.19 to 2.30 kg, 50 µL of the Gatifloxacin-containing eyedrops of each formulation were administered by instillation once. Two hours after instillation, rabbits were euthanized by excessive administration of 5% pentobarbital sodium. The anterior eye part was washed with physiological saline and the aqueous humor was collected by a syringe with a 27 gage injection needle.

The aqueous humor thus collected was filtered through a membrane filter (0.22 µm) to obtain a sample solution.

With respect to 50 µL of the sample solution, the concentration of free Gatifloxacin was measured under the same HPLC conditions as those in Test Example 1 (n = 3).

### (Results)

The results are shown in Table 12.

**Table 12**

| | Concentration of free Gatifloxacin in aqueous humor (µg/mL) | | | | |
|---|---|---|---|---|---|
| Formulation | Comparative Example 3 | Example 10 | Example 11 | Example 12 | Example 13 |
| average ± standard deviation | 1.13 ± 0.46 | 1.26 ± 0.44 | 1. 98 ± 0.73 | 2.60 ± 0.97 | 3.74 ± 1.60 |

As shown in Table 12, the eyedrops containing 0.1 to 0.5 w/v% xanthan gum of Examples 10 to 13 show increased concentrations of free Gatifloxacin in aqueous humor as compared with the eyedrops containing no xanthan gum of Comparative Example 3.

### Test Example 6: Low-temperature Storage Test and Freeze-Thawing Test

### (Preparation of Test Pharmaceutical Preparation)

To 2500 mL of purified water at 25°C was added 6. 25 g of xanthan gum with stirring. The mixture was warmed to 80°C, and was stirred for 5 hours with supplementing evaporated water every one hour. After completion of warming with stirring, the mixture was allowed to cool at room temperature. When the temperature of the mixture became 30°C of lower, water was added to make the total volume up to 2500 mL. The resulting solution was filtered through a membrane filter (5 µm) to obtain a 0.25% xanthan gum solution. To 1600 mL of this 0.25% xanthan were added 11 g of sodium chloride, 16 g of sodium dihydrogenphosphate dihydrate, 15 g of Gatifloxacin 3/2 hydrate and 40 mL of 0.5% sodium edetate solution and the mixture was dissolved. To the solution was added 50 mL of 0.2% benzalkonium chloride solution and the mixture was dissolved. To the solution was added purified water to make the total volume up to 1800 mL to prepare a Gatifloxacin-containing solution. Ninety mL of the Gatifloxacin-containing solution was measured, adjusted to pH 6, and made the total volume up to 100 mL by addition of purified water. The resulting solution was subjected to filtration sterilization with a membrane filter (0.22 µm) and 5 mL aliquots thereof were filled in polypropylene (PP) containers and polyethylene (PE) containers, respectively (Formulation 1). Further, 90 mL aliquots of the Gatifloxacin-containing solution were measured and the additives shown in Table 13 were added thereto so that Formulations 2 to 13 were obtained, followed by dissolving the mixtures. The resulting solution was adjusted to pH 6, made the total volume up to 100 mL by addition of purified water, and subjected to sterilized filtration with a membrane filter (0.22 µm). Five ml aliquots thereof were filled in polypropylene containers and polyethylene containers, respectively (Formulations 2 to 13).

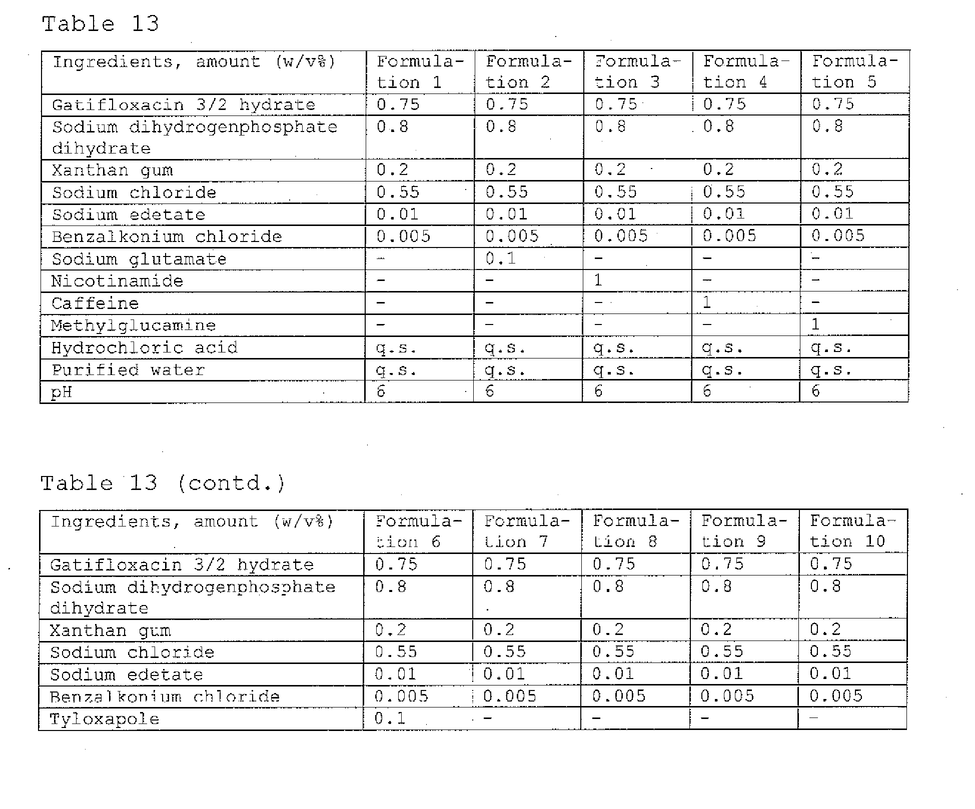

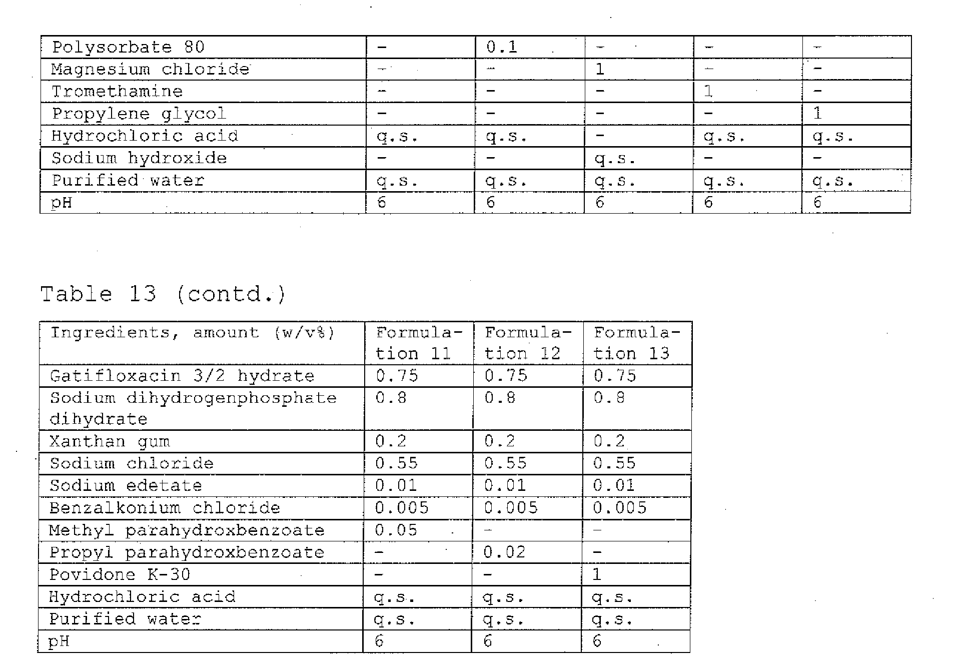

As xanthan gum, ECHO GUM T manufactured by Dainippon Sumitomo Pharma Co., Ltd. was used.

### (Test Procedure)

### (1) Low-Temperature (4°C) Storage Test

Four samples of each of the Gatifloxacin-containing eyedrops of respective Formulations were stored at 4°C for 4 weeks and the description of the samples were visually observed. In accordance with the criteria of Test Example 4, formation of a precipitate was judged and the number of (+) samples wherein the formation of foreign insoluble matter and description variation was observed was counted.

### (2) Freeze-Thawing Test

Three samples of each of the Gatifloxacin-containing eyedrops of respective Formulations were frozen at -30°C. Then, the samples were stored at 25°C to thaw the samples. These freeze-thawing procedures were repeated ten times and, after confirming that the sample completely thawed, the description was visually observed. In accordance with the criteria of Test Example 4, the formation of a precipitate was judged and the number of (+) samples wherein formation of foreign insoluble matter and description variation was observed was counted.

### (Results)

The results of the Law-Temperature (4°C) Test are shown in Table 14.

**Table 14**

| Formulation No. | Container | Sample number | Initial | After 4 week storage |
|---|---|---|---|---|
| Formulation 1 | PP | 4 | 0 | 2 |
| | PE | 4 | 0 | 2 |
| Formulation 2 | PP | 4 | 0 | 3 |
| | PE | 4 | 0 | 2 |
| Formulation 3 | PP | 4 | 0 | 0 |
| | PE | 4 | 0 | 0 |
| Formulation 4 | PP | 4 | 0 | 0 |
| | PE | 4 | 0 | 0 |
| Formulation 5 | PP | 4 | 0 | 0 |
| | PE | 4 | 0 | 0 |
| Formulation 6 | PP | 4 | 0 | 2 |
| | PE | 4 | 0 | 4 |
| Formulation 7 | PP | 4 | 0 | 0 |
| | PE | 4 | 0 | 0 |
| Formulation 8 | PP | 4 | 0 | 4 |
| | PE | 4 | 0 | 4 |
| Formulation 9 | PP | 4 | 0 | 0 |
| | PE | 4 | 0 | 0 |
| Formulation 10 | PP | 4 | 0 | 0 |
| | PE | 4 | 0 | 0 |
| Formulation 11 | PP | 4 | 0 | 0 |
| | PE | 4 | 0 | 0 |
| Formulation 12 | PP | 4 | 0 | 2 |
| Formulation 13 | PE | 4 | 0 | 1 |
| | PP | 4 | 0 | 3 |
| | PE | 4 | 0 | 3 |

The results of the Freeze-Thawing Test are shown in Table 15.

**Table 15**

| Formulation No. | Container | Sample number | Initial | After 10 times |
|---|---|---|---|---|
| Formulation 1 | PP | 3 | 0 | 1 |
| | PE | 3 | 0 | 1 |
| Formulation 2 | PP | 3 | 0 | 2 |
| | PE | 3 | 0 | 3 |
| Formulation 3 | PP | 3 | 0 | 0 |
| | PE | 3 | 0 | 0 |
| Formulation 4 | PP | 3 | 0 | 0 |
| | PE | 3 | 0 | 0 |
| Formulation 5 | PP | 3 | 0 | 0 |
| | PE | 3 | 0 | 0 |
| Formulation 6 | PP | 3 | 0 | 1 |
| | PE | 3 | 0 | 0 |
| Formulation 7 | PP | 3 | 0 | 2 |
| | PE | 3 | 0 | 3 |
| Formulation 8 | PP | 3 | 0 | 3 |
| | PE | 3 | 0 | 3 |
| Formulation 9 | PP | 3 | 0 | 3 |
| | PE | 3 | 0 | 3 |
| Formulation 10 | PP | 3 | 0 | 3 |
| | PE | 3 | 0 | 3 |
| Formulation 11 | PP | 3 | 0 | 0 |
| | PE | 3 | 0 | 0 |
| Formulation 12 | PP | 3 | 0 | 0 |
| | PE | 3 | 0 | 0 |
| Formulation 13 | PP | 3 | 0 | 3 |
| | PE | 3 | 0 | 2 |

As seen from the above results, it has been found that the formation of a precipitate during storage at a lower temperature (4°C) and at the time of freezing and thawing of the aqueous liquid preparation can be suppressed by addition of nicotinamide, caffeine, methylglucamine, methyl parahydroxybenzoate and a salt thereof to Gatifloxacin-containing eyedrops.

As described hereinabove, according to the present invention, intraocular penetration of Gatifloxacin in an aqueous liquid preparation comprising Gatifloxacin can be improved. Further, the formation of a precipitate during storage at a lower temperature and at the time of freezing and thawing of the aqueous liquid preparation can be suppressed.

## Claims

1. An aqueous liquid preparation comprising Gatifloxacin or a pharmacologically acceptable salt thereof or a hydrate thereof, phosphoric acid or a salt thereof, and xanthan gum, wherein a pH thereof is 5.5 or more and less than 7.0.

2. The aqueous liquid preparation according to claim 1, wherein the preparation contains 0.45 to 2 w/v% of Gatifloxacin or a pharmacologically acceptable salt thereof or a hydrate thereof as free Gatifloxacin.

3. The aqueous liquid preparation according to claim 1 or 2, wherein the preparation contains at least 0.6 w/v% or more of phosphoric acid or a salt thereof.

4. The aqueous liquid preparation according to claim 1 or 2, wherein the preparation contains 0.6 to 1.9 w/v% of phosphoric acid or a salt thereof.

5. The aqueous liquid preparation according to any one of claims 1 to 4, wherein the preparation further comprises sodium chloride.

6. The aqueous liquid preparation according to any one of claims 1 to 5, wherein the preparation further comprises at least one of the ingredient selected from the group consisting of nicotinamide, caffeine, methylglucamine, methyl parahydroxybenzoate and a salt thereof.

7. The aqueous solution according to any one of claims 1 to 6 which is eyedrops.

8. A method for suppressing the formation of a precipitate during storage at a lower temperature and at the time of freezing and thawing of an aqueous liquid preparation comprising Gatifloxacin or a pharmacologically acceptable salt thereof or a hydrate thereof, phosphoric acid or a salt thereof, and xanthan gum, whose pH is 5.5 or more and less than 7.0, which comprises incorporating at least one of the ingredient selected from the group consisting of nicotinamide, caffeine, methylglucamine, methyl parahydroxybenzoate and a salt thereof into the aqueous liquid preparation.
